(19) Europäisches Patentamt
European Patent Office
Office européen des brevets

(11) Veröffentlichungsnummer : **0 173 201**
**B1**

# (12) EUROPÄISCHE PATENTSCHRIFT

(45) Veröffentlichungstag der Patentschrift : **30.09.87**

(51) Int. Cl.⁴ : **C 07 C 17/22**, C 07 C 76/02, C 07 C 25/02, C 07 C 79/12

(21) Anmeldenummer : **85110344.0**

(22) Anmeldetag : **19.08.85**

## (54) Verfahren zur Herstellung aromatischer Bromverbindungen.

(30) Priorität : **30.08.84 DE 3431826**

(43) Veröffentlichungstag der Anmeldung : **05.03.86 Patentblatt 86/10**

(45) Bekanntmachung des Hinweises auf die Patenterteilung : **30.09.87 Patentblatt 87/40**

(84) Benannte Vertragsstaaten : **BE CH DE FR GB IT LI NL**

(56) Entgegenhaltungen :
**DE-B- 1 237 552**
**DE-B- 2 256 167**
**CHEMICAL ABSTRACTS, Band 66, Nr. 13, 27. März 1967, Columbus, Ohio, USA A.A. PANOMARENKO et al. "Direct replacement of the nitro group in aromatic and heterocyclic nitro compounds by halogen. VII. Substitution of the nitro group by bromine in nitrobenzene" Seite 5148, Zusammenfassung-Nr. 54 723u**
**JOURNAL OF THE CHEMICAL SOCIETY, TRANSACTIONS, Band 117, 1920, London S.N. DHAR "Experiments on halogenation. The direct displacement of negative groups by halogen in the aromatic series. Part I. The displacement of the nitro group by bromine" Seiten 993-1001**
**HOUBEN-YEYL "Methoden der organischen Chemie", Band V/4 "Halogenverbindungen" 1960 GEORG THIEME VERLAG, Stuttgart Seite 296**
**CESARE FERRI "Reaktionen der organischen Synthese" 1978 GEORG THIEME VERLAG, Stuttgart Seite 187**

(73) Patentinhaber : **HOECHST AKTIENGESELLSCHAFT**
**Postfach 80 03 20**
**D-6230 Frankfurt am Main 80 (DE)**

(72) Erfinder : **Metz, Hans Joachim, Dr.**
**Reiterweg 8**
**D-6148 Heppenheim (DE)**
Erfinder : **Warning, Klaus, Dr.**
**Kreuzheck 6**
**D-6239 Eppenstein/Taunus (DE)**

EP 0 173 201 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Aromatische Bromverbindungen wie z. B. Brombenzol oder 3,5-Dibrombenzoesäure, sind hauptsächlich wertvolle Zwischenprodukte auf verschiedenen Sachgebieten wie dem Farben-, Polymeren- und Pharmasektor.

Zu ihrer Herstellung ist eine Reihe verschiedener Verfahren bekannt. Ein übliches und häufig durchgeführtes Verfahren ist die Kernbromierung aromatischer Verbindungen, bei der jedoch fast immer — falls es nicht nur um die Monobromierung von (unsubstituiertem) Benzol geht — Isomerengemische erhalten werden. Die einzelnen Isomeren können oft nur mit erheblichem Aufwand getrennt werden. Bestimmte, speziell mehrfach kernbromierte Aromaten lassen sich — bedingt durch die dirigierende Wirkung bereits vorhandener Substituenten — nach diesem Verfahren kaum wirtschaftlich herstellen. Oft wird dann der Umweg über die sogenannte « Sandmeyer-Reaktion » gewählt.

Diese geht aus von aromatischen Aminen bzw. aromatischen Nitroverbindungen, die zu den Aminen reduziert werden. Das jeweilige Amin wird dann in das entsprechende Diazoniumsalz überführt, das schließlich in einer weiteren Stufe mit einem Bromid unter der katalytischen Wirkung von Kupfer-I-Ionen in das gewünschte Bromderivat überführt wird. Die mehrstufige Arbeitsweise sowie der Umstand, daß kupferhaltige Abwässer aufbereitet werden müssen, führen zu beträchtlichen Fertigungskosten.

Eine weitere, nur relativ wenig beachtete Möglichkeit zur Herstellung aromatischer Bromverbindungen besteht in dem direkten Ersatz der Nitrogruppen aromatischer Nitroverbindungen durch elementares Brom (S.N. Dhar, J. Chem. Soc. (1920), 117, 993). Das Verfahren arbeitet im allgemeinen bei Temperaturen bis zu etwa 250 °C — in speziellen Einzel-Fällen bis maximal 300 °C — im geschlossenen System, also wohl vorwiegend in flüssiger Phase. Es ist hierbei allerdings praktisch nicht möglich, selektiv nur die in den aromatischen Ausgangs-Nitroverbindungen vorhandenen Nitrogruppen durch Br zu ersetzen, ohne daß eine weitergehende Substitution der noch vorhandenen aromatisch gebundenen H-Atome erfolgt. Endprodukte der Reaktion sind daher in der Regel höher bromierte Produkte, d.h. aromatische Bromverbindungen, welche mehr Br-Substituenten als vorher Nitrogruppen besitzen. Der Grund für die Weiter-bromierung über den Ersatz der vorhandenen Nitrogruppen hinaus soll die Bildung eines reaktiven Br-haltigen Zwischenproduktes ($NO_2Br$) sein, welches leicht mit noch freien (unsubstituierten) Positionen des aromatischen Systems unter Substitution der H-Atome durch Br reagiert, z. B. :

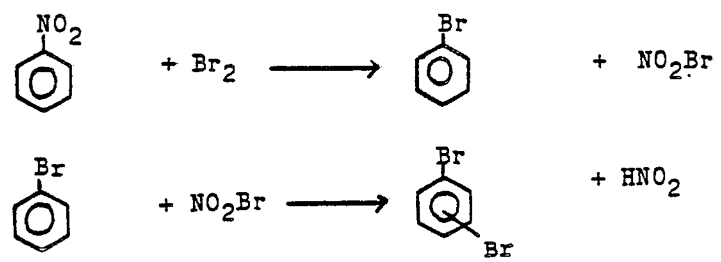

Wegen der meist unerwünschten Weiterbromierung der aromatischen Nitroverbindungen über den Ersatz der Nitrogruppen hinaus bestand die Aufgabe, das bekannte Verfahren so zu modifizieren, daß über den Ersatz der vorhandenen Nitrogruppen hinaus keine weitere Bromierung mehr stattfindet.

Diese Aufgabe konnte erfindungsgemäß durch Umsetzung in der Gasphase bei Temperaturen zwischen etwa 310 und 550 °C gelöst werden.

Erfindungsgegenstand ist daher ein Verfahren zur Herstellung aromatischer Bromverbindungen durch Umsetzung aromatischer Nitroverbindungen mit elementarem Brom bei erhöhter Temperatur, das dadurch gekennzeichnet ist, daß man die Umsetzung in der Gasphase bei Temperaturen zwischen etwa 310 und 550 °C durchführt.

Bei dieser Verfahrensweise wird ausschließlich an den Stellen des Moleküls ein Br-Substituent eingeführt, an denen zuvor die Nitrogruppe gebunden war. Dies ist außerordentlich überraschend, weil insbesondere aufgrund des von Dhar a.a.O. postulierten Mechanismus der bekannten Reaktion (über das reaktive Zwischenprodukt $NO_2Br$) davon auszugehen war, daß das reaktive Zwischenprodukt $NO_2Br$ unabhängig von den Reaktionsbedingungen entsteht und in jedem Fall eine Weiterbromierung der aromatischen Ausgangs-Nitroverbindungen über den Ersatz der vorhandenen Nitrogruppen durch Br hinaus stattfindet.

Für das erfindungsgemäße Verfahren können als Ausgangsverbindungen im Prinzip alle möglichen aromatischen Nitroverbindungen eingesetzt werden ; bevorzugt ist jedoch (wegen der nicht so hohen Zersetzlichkeit bei der Überführung in die Gasphase) der Einsatz von — gegebenenfalls durch inerte Gruppen substituiertem — Mono- oder Dinitrobenzol. Besonders bevorzugte Ausgangsverbindungen sind die Verbindungen der Formel I

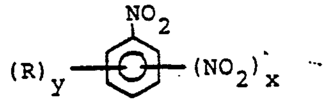

(I)

worin R = Halogen,
Trihalogenmethyl,
COOH,
CN,
COOR' (R' = niederes Alkyl),
COCl,
COBr,
$CONR''_2$ (R'' = H oder niederes Alkyl) ;
$OC_6H_5$,
$OC_nF_{2n+1}$ (n = ganze Zahl von 1-3),
NCO
x = 0 oder 1, und
y = 0 oder eine ganze Zahl von 1-5 (falls x = 0) bzw.
= 0 oder eine ganze Zahl von 1-4 (falls x = 1).

Ganz besonders sind diejenigen Verbindungen der Formel I bevorzugt, bei denen
R = F, Cl,
$CF_3$, $CCl_3$,
COOH,
CN,
COCl, COBr,
x = 0 oder 1 und
y = 0 oder 1.

Unter die Formel I fallende beispielhafte Ausgangsverbindungen sind :

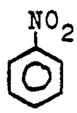 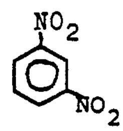 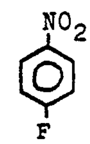 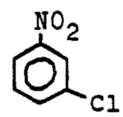

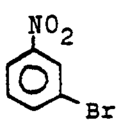 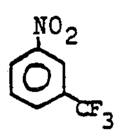 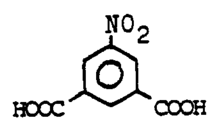 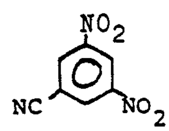 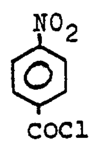

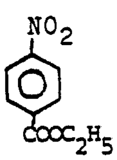 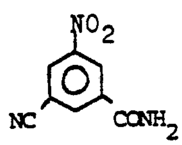 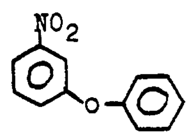 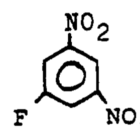 ,etc.

Die Verbindungen sind nach bekannten Verfahren zugänglich.

Das Molverhältnis von aromatischer Ausgangs-Nitroverbindung : elementarem Brom ist nicht besonders kritisch ; bevorzugt wird jedoch pro Mol der in den aromatischen Nitroverbindungen vorhandenen Nitrogruppen etwa 1/2 bis 1 Mol elementares Brom verwendet. Das Brom kann je nach Bedarf rein oder mit einem Inertgas (z. B. $N_2$, Argon, etc.) vermischt und in die Reaktion eingesetzt werden.

Zur Durchführung des Verfahrens wird die aromatische Nitroverbindung verdampft und in der Gasphase mit Bromdämpfen bei den vorstehend angegebenen Temperaturen (zwischen etwa 310 und 550 °C) in Kontakt gebracht. Die Verdampfung der aromatischen Nitroverbindungen kann — je nach deren Stabilität und den technischen Gegebenheiten — bei vermindertem, bei erhöhtem oder bei Normaldruck durchgeführt werden, wobei die spezielle Verdampfungstemperatur in bekannter Weise nach dem jeweils eingestellten Druck zu wählen ist. Bevorzugter Druck ist Normaldruck.

Die Verdampfung kann durch Zusatz eines Inertgases, das als Schlepper dient, unterstützt werden. Die verdampfte aromatische Nitroverbindung wird anschließend mit den Bromdämpfen vermischt und bei der gewünschten Temperatur innerhalb des angegebenen Bereichs zur Reaktion gebracht. Die optimale Reaktionstemperatur richtet sich nach der Natur der eingesetzten aromatischen Nitroverbindung und ist gegebenenfalls durch einfache Vorversuche zu ermitteln.

Das Verfahren kann vorzugsweise in einem stationär betriebenen Strömungsrohr durchgeführt werden, in dem die verdampfte aromatische Nitroverbindung zusammen mit der gewünschten Menge Brom auf die jeweilige Reaktionstemperatur aufgeheizt wird. Es ist möglich, die Reaktion im gesamten

Druckbereich durchzuführen, in dem das Reaktionsgemisch bei der jeweiligen Temperatur noch gasförmig ist.

Die optimalen Verweilzeiten liegen normalerweise im Bereich von etwa 20 bis 400 Sekunden und werden gegebenenfalls zusammen mit der optimalen Reaktionstemperatur in einfachen Vorversuchen ermittelt.

Die Auftrennung der erhaltenen Reaktionsgemische geschieht zweckmäßig entweder nur durch Rektifikation oder durch Auswaschen der anorganischen Nebenprodukte z. B. mit Sulfit/Carbonat- oder einer Alkalihydroxid-Lösung, und nachgeschalteter Rektifikation der organischen Produkte.

Nicht umgesetztes Ausgangsmaterial wird nach seiner — vorzugsweise destillativen — Abtrennung erneut eingesetzt.

Wegen der Eröffnung der Möglichkeit des selektiven und gezielten Ersatzes von Nitrogruppen in aromatischen Nitroverbindungen durch Br — ohne darüber hinausgehende weitere Substitution durch Br — stellt die Erfindung eine wesentliche Bereicherung der Technick auf dem Gebiet der Herstellung bromhaltiger Aromaten dar.

Die Erfindung wird nun durch die folgenden Beispiele näher erläutert.

## Beispiele

1. Herstellung von 4-Bromfluorbenzol

141 g (= Mol) 4-Fluornitrobenzol wurden bei 200-240 °C verdampft und zusammen mit 128 g (= 0,8 Mol) Brom (Molverhältnis 1 : 0,8) im Laufe von 4 Stunden durch ein Strömungsrohr (Dimension : Ø = 30 mm, 1 = 600 mm) geleitet ; der Gasstrom wurde dabei auf eine Endtemperatur von 400 °C aufgeheizt. Der organische Teil des Produktgemisches zeigte folgende Zusammensetzung (GC-Analyse) :

| | |
|---|---|
| 4-Bromfluorbenzol | 62 % |
| 4-Fluornitrobenzol | 35 % |
| Nebenprodukte | 3 % |

Die Ausbeute an 4-Bromfluorbenzol, bezogen auf umgesetztes 4-Fluornitrobenzol, betrug demnach 85,5 % d. Th.

2. Herstellung von 1,3-Dibrombenzol

200 g (= 0,99 Mol) 3-Bromnitrobenzol und 126 g (= 0,78 Mol) Brom (Molverhältnis 1 : 0,8) wurden gemeinsam verdampft und während 4 Stunden durch ein Strömungsrohr bei $T_{max}$ = 420 °C geleitet. Das Gemisch der organischen Reaktionsprodukte zeigte folgende Zusammensetzung (GC-Analyse) :

| | |
|---|---|
| 1,3-Dibrombenzol | 77 % |
| 3-Bromnitrobenzol | 16 % |
| Nebenprodukte | 7 % |

Die Ausbeute an 1,3-Dibrombenzol, bezogen auf umgesetztes 3-Bromnitrobenzol, betrug demnach 85,6 % d. Th.

3. Herstellung von 1,3-Dibrombenzol und von 3-Bromnitrobenzol

104 g (= 0,62 Mol) 1,3-Dinitrobenzol wurden zusammen mit 120 g (= 0,75 Mol) Brom im Stickstoffstrom (5 1/h) verdampft und im Laufe von 3 Stunden durch ein Strömungsrohr bei $T_{max}$ = 350 °C geleitet. Es wurde ein Produktgemisch folgender Zusammensetzung (GC-Analyse) erhalten :

| | |
|---|---|
| 1,3-Dibrombenzol | 28 % |
| 3-Bromnitrobenzol | 31 % |
| 1,3-Dinitrobenzol | 39 % |
| Nebenprodukte | 2 % |

Die Ausbeute an 1,3-Dibrombenzol, bezogen auf umgesetztes 1,3-Dinitrobenzol, betrug demnach 31,7 % d. Th. Die Ausbeute an 3-Bromnitrobenzol, bezogen auf umgesetztes 1,3-Dinitrobenzol, betrug 41,0 % d. Th.

**Patentansprüche**

1. Verfahren zur Herstellung aromatischer Bromverbindungen durch Umsetzung aromatischer

Nitroverbindungen mit elementarem Brom bei erhöhter Temperatur, dadurch gekennzeichnet, daß man die Umsetzung in der Gasphase bei Temperaturen zwischen etwa 310 und 550 °C durchführt.

2. Verfahren nach Anspruch 1, dadurch gekennzeichnet, daß man als aromatische Nitroverbindungen gegebenenfalls durch inerte substituiertes Mono- oder Dinitrobenzol verwendet.

3. Verfahren nach Anspruch 1 oder 2, dadurch gekennzeichnet, daß man als aromatische Nitroverbindungen Verbindungen der Formel I

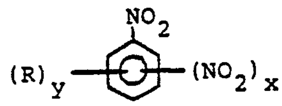

(I)

worin R = F, Cl, Br
Trifluormethyl,
CN,
COBr,
$OC_6H_5$,
$OC_nF_{2n+1}$ (n = ganze Zahl von 1-3),
NCO,
x = 0 oder 1, und
y = 0 oder eine ganze Zahl von 1-5 (falls x = 0) bzw.
= 0 oder eine ganze Zahl von 1-4 (falls x = 1)
verwendet.

4. Verfahren nach Anspruch 3, dadurch gekennzeichnet, daß man als aromatische Nitroverbindungen Verbindungen der Formel I mit
R = F, Cl, Br,
$CF_3$,
CN,
COBr
x = 0 oder 1 und
y = 0 oder 1
verwendet.

5. Verfahren nach einem oder mehreren der Ansprüche 1 bis 4, dadurch gekennzeichnet, daß man pro Mol Nitrogruppen in den aromatischen Ausgangs-Nitroverbindungen etwa 1/2 bis 1 Mol Brom verwendet.

6. Verfahren nach einem oder mehreren der Ansprüche 1 bis 5, dadurch gekennzeichnet, daß man die verdampfte aromatische Nitroverbindung mit Brom durch ein auf mindestens 310 °C erhitztes Strömungsrohr führt.

7. Verfahren nach Anspruch 6, dadurch gekennzeichnet, daß die Verweilzeit des Gasgemisches etwa 20-400 Sekunden beträgt.

**Claims**

1. A process for preparing aromatic bromine compounds by reacting aromatic nitro compounds with elemental bromine at elevated temperature, characterized in that the reaction is carried out in the gas phase at temperatures between about 310 and 550 °C.

2. Process as claimed in claim 1, characterized in that the aromatic nitro compounds used are mononitrobenzene or dinitrobenzene which may be substituted by inert groups.

3. Process as claimed in claim 1 or 2, characterized in that the aromatic nitro compounds used are compounds of the formula I

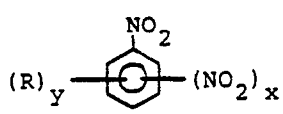

(I)

in which R = F, Cl, Br,
trifluoro,
CN,
COBr,
$OC_6H_5$,
$OC_nF_{2n+1}$ (n = integer from 1-3),
NCO,
x = 0 or 1, and

y = 0 or an integer from 1-5 (if x = 0) or
= 0 or an integer from 1-4 (if x = 1).

4. Process as claimed in claim 3, characterized in that the aromatic nitro compounds used are compounds of the formula I where

R = F, Cl, Br,
$CF_3$,
CN,
COBr,
x = 0 or 1 and
y = 0 or 1.

5. Process as claimed in one or more of claims 1 to 4, characterized in that about 1/2 to 1 mol of bromine is used per mol of nitro groups in the aromatic starting nitro compounds.

6. Process as claimed in one or more of claims 1 to 5, characterized in that the vaporized aromatic nitro compound is conducted with bromine through a flow-pipe which is heated to at least 310 °C.

7. Process as claimed in claim 6, wherein the residence time of the gaseous mixture is in the range from 20 to 400 seconds.

**Revendications**

1. Procédé de préparation de composés aromatiques bromés par réaction de composés aromatiques nitrés avec du brome élémentaire à une température élevée, procédé caractérisé en ce que l'on effectue la réaction en phase gazeuse à des températures comprises entre environ 310 et 550 °C.

2. Procédé selon la revendication 1 caractérisé en ce que l'on utilise comme composés aromatiques nitrés du mononitrobenzène ou du dinitrobenzène qui peuvent avoir éventuellement des groupes inertes.

3. Procédé selon la revendication 1 ou 2 caractérisé en ce que l'on utilise comme composés aromatiques nitrés des composés de formule I ci-dessous :

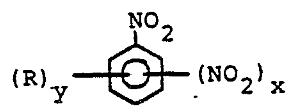

(I)

dans laquelle R = F, Cl, Br
le groupe trifluorométhyle,
CN,
COBr,
$OC_6H_5$,
$OC_nF_{2n+1}$ (n étant un entier de 1 à 3),
NCO,
x = 0 ou 1, et
y = 0 ou un entier de 1 à 5 (si x = 0) ou bien
= 0 ou un entier de 1 à 4 (si x = 1).

4. Procédé selon la revendication 3 caractérisé en ce que l'on utilise comme composés aromatiques nitrés des composés de formule I dans lesquels :

R = F, Cl, Br,
$CF_3$,
CN,
COBr,
x = 0 ou 1, et
y = 0 ou 1.

5. Procédé selon une ou plusieurs des revendications 1 à 4, caractérisé en ce que l'on utilise, par mole de groupes nitro des composés aromatiques nitrés, environ 1/2 à 1 mole de brome.

6. Procédé selon une ou plusieurs des revendications 1 à 5, caractérisé en ce que l'on fait passer la vapeur du composé aromatique nitré avec la vapeur de brome dans un réacteur tubulaire à écoulement chauffé à 310 °C au moins.

7. Procédé selon la revendication 6 caractérisé en ce que le temps de séjour du mélange gazeux dans le réacteur est d'environ 20 à 400 secondes.